(19) Europäisches Patentamt
European Patent Office
Office européen des brevets

(11) Numéro de publication : **0 336 803 B1**

(12) # FASCICULE DE BREVET EUROPEEN

(45) Date de publication du fascicule du brevet : **30.09.92 Bulletin 92/40**

(51) Int. Cl.$^5$ : **C11D 9/26**

(21) Numéro de dépôt : **89400749.1**

(22) Date de dépôt : **17.03.89**

(54) **Savon transparent contenant un alcanediol-1,2.**

(30) Priorité : **24.03.88 LU 87179**

(43) Date de publication de la demande : **11.10.89 Bulletin 89/41**

(45) Mention de la délivrance du brevet : **30.09.92 Bulletin 92/40**

(84) Etats contractants désignés : **AT BE CH DE ES FR GB GR IT LI NL SE**

(56) Documents cités :
**FR-A- 2 125 530**
**GB-A- 1 300 415**
**US-A- 4 165 293**

(73) Titulaire : **L'OREAL**
**14, Rue Royale**
**F-75008 Paris (FR)**

(72) Inventeur : **Verite, Claude**
**4, rue Jacques Coeur**
**F-75004 Paris (FR)**
Inventeur : **Caudet, Alain**
**255 boulevard Jean-Jaurès**
**F-92100 Boulogne-Billancourt (FR)**

(74) Mandataire : **Casalonga, Axel et al**
**BUREAU D.A. CASALONGA - JOSSE**
**Morassistrasse 8**
**W-8000 München 5 (DE)**

**EP 0 336 803 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une composition de savon solide, transparente à base de savons d'acides gras de suif comprenant au moins un alcanediol-1,2.

Les compositions de savons solides, transparentes, sont bien connues dans l'état de la technique. Elles sont généralement constituées, avec ou sans alcool, à partir de savons d'acides gras de suif et/ou de coprah et/ou de ricin et d'agents de transparence choisis de préférence parmi les polyols tels que les sucres, la glycérine ou le glycols tels que le propylène glycol, l'éthylène glycol ou des mélanges de ces derniers.

Dans le cas des savons solides sans alcool on utilise généralement une teneur en savon inférieure à 40% et notamment inférieure à 20% en savons d'acides gras de coprah pour améliorer leur transparence.

Ces compositions connues dans le domaine cosmétique présentent l'inconvénient d'être peu moussantes. On a cherché à améliorer leur pouvoir moussant par l'addition d'agents tensio-actifs anioniques de synthèse tels que les oléfines sulfonates de métaux alcalins.

On connaît également des compositions de savons solides sans alcool du même type, contenant également un agent synergiste de mousse comme le diéthanolamide de coprah.

La demanderesse a découvert d'une manière surprenante qu'en introduisant un alcanediol-1,2 dans une composition de savon solide transparent, sans alcool, à base de savons d'acides gras, on améliorait sensiblement les qualités de mousse de la composition, en particulier son abondance, sa texture, sa douceur et sa rapidité de développement.

Les compositions selon l'invention présentent des qualités de mousse supérieures à celles obtenues avec le diéthanolamide de coprah utilisé antérieurement et de façon conventionnelle ou par rapport à un autre agent synergiste de mousse tel que l'alkyl (coprah) amido propyldiméthyl glycine vendu sous la dénomination "TEGO BETAIN L7" par la société GOLDSCHMIDT.

La présente invention a donc pour objet une composition cosmétique détergente et moussante se présentant sous forme de savon solide, transparent à base de savons d'acides gras de suif, d'eau, d'agents de transparence et contenant au moins un alcanediol-1,2.

Un autre objet de l'invention est un procédé de lavage mettant en oeuvre la composition définie ci-dessus.

D'autres objets de l'invention apparaîtront à la lecture de la description et des exemples qui suivent.

La composition conforme à l'invention se présente sous forme d'un pain solide transparent, essentiellement caractérisée par le fait qu'elle contient un savon constitué de sels d'acides gras en $C_{10}$-$C_{20}$, un alcanediol-1,2, un agent de transparence et de l'eau dans des quantités inférieures ou égales à 25% du poids total de la composition.

Les alcanediol-1,2, utilisables dans la présente invention sont de composés saturés avec une chaîne linéaire à nombre pair ou impair d'atomes de carbone pouvant comporter de 10 à 18 et de préférence de 10 à 14 atomes de carbone. Le dodécanediol-1,2 est l'alcanediol-1,2 préféré. Ils peuvent être obtenus commodément par hydroxylation des $\alpha$-oléfines ou hydrolyse de époxydes correspondants.

On utilise de préférence, l'alcanediol-1,2 dans des proportions pondérales comprises entre 3 et 10%, de préférence entre 4 et 8% et plus particulièrement encore entre 5 et 7% par rapport au poids total de la composition.

Les savons utilisés selon l'invention, sont choisis de préférence parmi les sels de sodium en particulier parmi les sels de sodium d'acides gras en $C_{16}$-$C_{20}$ et les sels de sodium d'acides gras en $C_{10}$-$C_{14}$ ou leur mélange. Ils sont utilisés préférentiellement dans des proportions comprises entre 25 et 40%, en particulier entre 25 et 35% par rapport au poids total de la composition. Des résultats particulièrement intéressants sont obtenus pour des proportions comprises entre 27 et 32% en poids.

Une forme de réalisation préférée de l'invention consiste à utiliser un savon renfermant une quantité pondérale de sels de sodium d'acides gras en $C_{16}$-$C_{20}$ comprise entre 80 et 90% et une quantité pondérale de sels de sodium d'acides gras en $C_{10}$-$C_{14}$ comprise entre 10 et 20%.

L'agent de transparence, qui est également un solvant, est choisi parmi les polyols en $C_2$-$C_6$ tels que plus particulièrement la glycérine, le propylène glycol, et le sorbitol ou éventuellement l'urée ainsi que leurs mélanges.

Il est utilisé de préférence dans des proportions comprises entre 25 et 50%.

Une composition de savon solide, transparente préférée est constituée par un pain comprenant :

(i) 25 à 40% d'un savon constitué par un mélange de sels de sodium d'acides gras en $C_{16}$-$C_{20}$ et de sels de sodium d'acides gras en $C_{10}$-$C_{14}$;

(ii) 3 à 10% de dodécanediol-1,2;

(iii) 25 à 50% d'un polyol en $C_2$-$C_6$ ou d'urée;

(iv) de l'eau dans des proportions inférieures ou égales à 25% en poids.

La présence de l'alcanediol-1,2 dans les compositions de savon solide transparent selon l'invention, modifie de façon importante par un effet de synergie leurs propriétés moussantes ainsi que l'aspect de la mousse en résultant.

On constate en effet : une très nette amélioration du démarrage de la mousse, une forte augmentation du pouvoir moussant; une amélioration de l'aspect de la mousse qui est plus épaisse, plus consistante, plus onctueuse donc plus agréable au toucher; la stabilité de la mousse est également accrue.

En plus de ses propriétés détergentes et moussantes, le savon utilisé conformément à la présente invention présente l'avantage d'être non irritant pour la peau.

Les compositions de savons solides et transparentes définies ci-dessus peuvent contenir en plus un agent tensio-actif anionique ou non ionique de synthèse choisi parmi l'oléfine ($C_{14}$-$C_{16}$) sulfonate de sodium, le laurylsulfate de triéthanolamine, le N-lauroylsarcosinate de sodium vendu sous les dénominations "SARKOSYL NL 30" par la société CIBA GEIGY ou "ORAMIX L 30" par la société SEPPIC, le sel de potassium d'un condensat d'acides gras du coprah et d'une protéine animale hydrolysée vendu sous la dénomination "LAMEPON S" par la société GRUNAU, un éther de glucose et d'alcool décylique vendu à 50% de matière active (MA) sous la dénomination "TRITON CG 312" par la société SEPPIC, ou un alkyl ($C_9$-$C_{18}$ linéaire ou ramifié) éther sulfate de métal alcalin comportant 1 à 10 moles d'oxyde d'éthylène tel qu'un alkyl ($C_{12}$-$C_{14}$ linéaire) éther sulfate de sodium à environ 2,2 moles d'oxyde d'éthylène, ou un alkyl ($C_{13}$-$C_{15}$ ramifié) éther sulfate de sodium à 3 moles d'oxyde d'éthylène vendu par la Société ICI sous la dénomination TENSAGEX DLM 970.

Lorsqu'ils sont présents ces agents tensio-actifs anioniques ou non ioniques sont utilisés dans les compositions selon l'invention dans des proportions comprises entre 1 et 10% et de préférence entre 4 et 8% par rapport au poids total de la composition.

Elles peuvent également contenir des agents sequestrants ou chélatants, tels que le sel tétrasodique de l'acide éthylène diamino tétraacétique, le sel tétrasodique de l'acide 1-hydroxyéthylidène diphosphonique. Ils sont utilisés dans des proportions de 0,1 à 0,5 % en poids par rapport au poids total de la composition.

Les compositions de savons solides, transparentes, selon l'invention peuvent renfermer également des adjuvants ne modifiant pas leur transparence tels que des agents nacrants et des silicones non volatils, dans une teneur pondérale inférieure à 5%, des agents de conditionnement de la peau tels que des polymères, des extraits d'aloes ou de mauve, de l'huile de tournesol ou du collagène dans une teneur pondérale inférieure à 2%. Les compositions conformes à l'invention peuvent également contenir des ingrédients actifs pour le traitement de la peau tels que des agents anti-acnéiques, des agents anti-bactériens; elles peuvent également renfermer des colorants et d'autres adjuvants habituellement utilisés en cosmétique.

Un autre objet de l'invention est un procédé de lavage de la peau caractérisé par le fait que l'on applique sur la peau humide un savon solide transparent tel que défini ci-dessus.

Les exemples suivants illustrent l'invention sans toutefois la limiter.

## EXEMPLE 1

On prépare un savon solide transparent de composition suivante :

| | | | |
|---|---|---|---|
| - Acide stéarique | | 22 | g |
| - Acide laurique | | 3 | g |
| - Hydroxyde de sodium | | 3,8 | g |
| - Glycérine | | 22 | g |
| - Propylène glycol | | 22 | g |
| - Dodécanediol-1,2 | | 7 | g |
| - Sel tétrasodique de l'acide éthylène diamino tétraacétique | | 0,2 | g |
| - Sel tétrasodique de l'acide 1-hydroxyéthylidène diphosphonique | | 0,1 | g |
| - Colorant, parfum | qs | | |
| - Eau | qsp | 100 | g |

EXEMPLE 2

On prépare un savon solide transparent de composition suivante :

| | | |
|---|---|---|
| - Acide stéarique | | 24,2 g |
| - Acide laurique | | 3,3 g |
| - Hydroxyde de sodium | | 4,2 g |
| - Glycérine | | 25 g |
| - Propylène glycol | | 25 g |
| - Dodécanediol-1,2 | | 5,5 g |
| - Sel tétrasodique de l'acide éthylène diamino tétraacétique | | 0,2 g |
| - Sel tétrasodique de l'acide 1-hydroxyéthylidène diphosphonique | | 0,1 g |
| - Colorant, parfum | qs | |
| - Eau | qsp | 100 g |

EXEMPLE 3

On prépare un savon solide transparent de composition suivante :

| | | |
|---|---|---|
| - Acide stéarique | | 22 g |
| - Acide laurique | | 3 g |
| - Hydroxyde de sodium | | 3,8 g |
| - Glycérine | | 24 g |
| - Propylène glycol | | 20 g |
| - Dodécanediol-1,2 | | 6 g |
| - Oléfine ($C_{14}$-$C_{16}$) sulfonate de sodium vendue à 37% de matière active (MA) sous la dénomination "ELFAN OS46" par la société AKZO | | 4 g MA |
| - Sel tétrasodique de l'acide éthylène diamino tétraacétique | | 0,2 g |
| - Sel tétrasodique de l'acide 1-hydroxyéthylidène diphosphonique | | 0,1 g |
| - Colorant, parfum | qs | |
| - Eau | qsp | 100 g |

EXEMPLE 4

On prépare un savon solide transparent de composition suivante :

| | | |
|---|---|---|
| - Acide stéarique | | 22,5 g |
| - Acide laurique | | 3,5 g |
| - Hydroxyde de sodium | | 4,0 g |
| - Glycérine | | 19 g |
| - Propylène glycol | | 21 g |
| - Dodécanediol-1,2 | | 7 g |
| - Sel de potassium du condensat d'acides gras de coprah et d'une protéine animale hydrolysée vendue à 30% MA sous la dénomination "LAMEPON S" par la société GRUNAU | | 6 g MA |
| - Sel tétrasodique de l'acide éthylène diamino tétracétique | | 0,2 g |
| - Sel tétrasodique de l'acide 1-hydroxyéthylidène diphosphonique | | 0,1 g |
| - Colorant, parfum | qs | |
| - Eau | qsp | 100 g |

EXEMPLE 5

On prépare un savon solide transparent de composition suivante :

| | | |
|---|---|---|
| - Acide stéarique | | 22 g |
| - Acide laurique | | 3 g |
| - Hydroxyde de sodium | | 3,8 g |
| - Glycérine | | 22 g |
| - Propylène glycol | | 22 g |
| - Dodécanediol-1,2 | | 6 g |
| - Ether de glucose et d'alcool décylique vendu à 50% de matière active (MA) sous la dénomination "TRITON CG 312" par la société SEPPIC | | 6,4 g MA |
| - Sel tétrasodique de l'acide éthylène diamine tétraacétique | | 0,2 g |
| - Sel tétrasodique de l'acide 1-hydroxyéthylidène diphosphonique | | 0,1 g |
| - Colorant, parfum | qs | |
| - Eau | qsp | 100 g |

EXEMPLE 6

On obtient un savon solide transparent en remplaçant dans l'exemple 3, les 6 g de dodécanediol-1,2 par 6 g de tétradécanediol-1,2.

EXEMPLE 7

On obtient un savon solide transparent en remplaçant dans l'exemple 3, les 6 g de dodécanediol 1,2 par 6 g de décanediol-1,2.

EXEMPLE 8

On obtient un savon solide transparent en reproduisant l'exemple 3, par remplacement des 4 g d' Elfan 0S46 par 6 g (MA) d'un alkyl ($C_{12}$-$C_{14}$ linéaire) éther sulfate de sodium à 2,2 moles d'oxyde d'éthylène vendu à 70% de matière active (MA).

Exemple 9

On prépare un savon solide transparent de composition suivante :

| | | | |
|---|---|---|---|
| - Acide stéarique | | 19,85 | g |
| - Acide laurique | | 3,50 | g |
| - Hydroxyde de sodium | | 3,75 | g |
| - Glycérine | | 8 | g |
| - Sorbitol à 70% (MA) en solution aqueuse | | 9 | g MA |
| - Propylèneglycol | | 20 | g |
| - Dodécanediol-1,2 | | 6 | g |
| - Alkyl ($C_{13}$-$C_{15}$) ramifié, éther sulfate de sodium à 3 moles d'oxyde d'éthylène vendu sous la dénomination "TENSAGEX DLM 970" par la Société ICI | | 6 | g MA |
| - Sel tétrasodique de l'acide éthylène diaminotétraacétique | | 0,2 | g |
| - Sel tétrasodique de l'acide 1-hydroxyéthylène diphosphonique | | 0,1 | g |
| - Colorant, parfum | qs | | |
| - Eau | qsp | 100 | g |

**Revendications**

**Revendications pour les Etats Contractants suivants : FR, BE, AT, GB, GR, IT, NL, DE, SE, CH, LI**

1. Composition sous forme d'un pain solide transparent, caractérisée par le fait qu'elle contient un savon constitué de sels d'acides gras en $C_{10}$-$C_{20}$, un alcanediol-1,2 comportant de 10 à 18 atomes de carbone, un agent de transparence, et de l'eau en des quantités inférieures ou égales à 25% par rapport au poids total de la composition.

2. Composition selon la revendication 1, caractérisée par le fait que l'alcanediol-1,2 comporte de 10 à 14 atomes de carbone.

3. Composition selon la revendication 1, caractérisée par le fait que l'alcanediol-1,2 est le dodécanediol-1,2.

4. Composition selon l'une quelconque des revendications 1 à 3, caractérisée par le fait que le l'alcanediol-1,2 est présent dans des proportions comprises entre 3 et 10% et de préférence entre 4 et 8% par rapport au poids total de la composition.

5. Composition selon la revendication 1, caractérisée par le fait que le savon est constitué par des sels de sodium d'acides gras en $C_{16}$-$C_{20}$ ou des sels de sodium d'acides gras en $C_{10}$-$C_{14}$ ou leurs mélanges.

6. Composition selon l'une quelconque des revendications 1 à 5, caractérisée par le fait que le savon est présent dans des proportions comprises entre 25 et 40% en poids par rapport au poids total de la composition et de préférence entre 25 et 35%.

7. Composition selon la revendication 5 ou 6, caractérisée par le fait que le savon est constitué de 80 à 90% de sels de sodium d'acides gras en $C_{16}$-$C_{20}$ et de 10 à 20% de sels de sodium d'acides gras en $C_{10}$-$C_{14}$ en poids par rapport à la quantité totale de savon.

8. Composition selon l'une quelconque des revendications 1 à 7, caractérisée par le fait que l'agent de transparence est choisi parmi les polyols en $C_2$-$C_6$, l'urée ainsi que leurs mélanges.

9. Composition selon la revendication 8, caractérisée par le fait que le polyol est la glycérine, le propylène glycol, ou le sorbitol ou leurs mélanges.

10. Composition selon l'une quelconque des revendications 1 à 9, caractérisée par le fait que l'agent de transparence est présent dans des proportions de 25 à 50% en poids par rapport au poids total de la composition.

11. Composition selon l'une quelconques des revendications 1 à 10, caractérisée par le fait qu'elle contient :
    (i) 25 à 40% d'un savon constitué par un mélange de sels de sodium d'acides gras en $C_{16}$-$C_{20}$ et de sels de sodium d'acides gras en $C_{10}$-$C_{14}$;
    (ii) 3 à 10% de dodécanediol-1,2;
    (iii) 25 à 50% d'un polyol en $C_2$-$C_6$ ou d'urée;
    (iv) de l'eau dans des proportions inférieures ou égales à 25% en poids.

12. Composition selon l'une quelconque des revendications 1 à 11, caractérisée par le fait qu'elle contient en plus un agent tensio-actif anionique ou non-ionique de synthèse dans des proportions comprises entre 1 et 10% et de préférence entre 4 et 8% par rapport au poids total de la composition.

13. Composition selon la revendication 12, caractérisée par le fait que l'agent tensio-actif est choisi parmi les oléfines ($C_{14}$-$C_{16}$) sulfonates de sodium, le lauryl sulfate de triéthanolamine, le N-lauroylsarcosinate de sodium, un sel de potassium d'un condensat d'acides gras du coprah et d'une protéine hydrolysée, un éther de glucose et d'alcool décylique, un alkyl ($C_9$-$C_{18}$ linéaire ou ramifié) éther sulfate de sodium comportant 1 à 10 moles d'oxyde d'éthylène.

14. Composition selon l'une quelconque des revendications 1 à 11, caractérisée par le fait qu'elle contient des agents sequestrants.

15. Composition selon l'une quelconque des revendications 1 à 14, caractérisée par le fait qu'elle contient des adjuvants choisis parmi les agents nacrants et les silicones non volatils, des agents de conditionnement de la peau, des extraits d'aloès ou de mauve, de l'huile de tournesol, du collagène, ou des colorants.

16. Composition selon l'une quelconque des revendication 1 à 15, caractérisée par le fait qu'elle contient des ingrédients actifs pour le traitement de la peau.

17. Utilisation de la composition telle que revendiquée dans les revendications 1 à 16 pour le lavage de la peau.

**Revendications pour l'Etat contractant suivant: ES**

1. Composition sous forme d'un pain solide transparent, caractérisé par le fait qu'elle contient :

(i) 25 à 40% en poids d'un savon constitué de sels d'acides gras en $C_{10}$-$C_{20}$ et de préférence 25% à 35% en poids par rapport au poids total de la composition,
(ii) 3 à 10% en poids d'un alcanediol-1,2 comportant de 10 à 18 atomes de carbone et de préférence 4 à 8% en poids.
(iii) 25 à 50% en poids d'un agent de transparence,
(iv) de l'eau dans des proportions inférieures ou égale à 25% en poids.

2. Composition selon la revendication 1, caractérisée par le fait que l'alcanediol-1,2 comporte de 10 à 14 atomes de carbone.

3. Composition selon la revendication 1, caractérisée par le fait que l'alcanediol-1,2 est le dodécanediol-1,2.

4. Composition selon la revendication 1, caractérisée par le fait que le savon est constitué par des sels de sodium d'acides gras en $C_{16}$-$C_{20}$ ou des sels de sodium d'acides gras en $C_{10}$-$C_{14}$ ou leurs mélanges.

5. Composition selon la revendication 4, caractérisée par le fait que savon est constitué de 80 à 90% de sels de sodium d'acides gras en $C_{16}$-$C_{20}$ et de 10 à 20% de sels de sodium d'acides gras en $C_{10}$-$C_{14}$ en poids par rapport à la quantité totale de savon.

6. Composition selon l'une quelconque des revendications 1 à 5, caractérisée par le fait que l'agent de transparence est choisi parmi les polyols en $C_2$-$C_6$, l'urée ainsi que leurs mélanges.

7. Composition selon la revendication 6, caractérisée par le fait que le polyol est la glycérine ou le propylène glycol, ou le sorbitol ou leurs mélanges.

8. Composition selon l'une quelconque des revendications 1 à 7, caractérisée par le fait que qu'elle contient :
(i) 25 à 40% d'un savon constitué par un mélange de sels de sodium d'acides gras en $C_{16}$-$C_{20}$ et de sels de sodium d'acides gras en $C_{10}$-$C_{14}$;
(ii) 3 à 10% de dodécanediol-1,2;
(iii) 25 à 50% d'un polyol en $C_2$-$C_6$ ou d'urée;
(iv) de l'eau dans des proportions inférieures ou égales à 25% en poids.

9. Composition selon l'une quelconque des revendications 1 à 8, caractérisée par le fait qu'elle contient en plus un agent tensio-actif anionique ou non-ionique de synthèse dans des proportions comprises entre 1 et 10% et de préférence entre 4 et 8% par rapport au poids total de la composition.

10. Composition selon la revendication 9, caractérisée par le fait que l'agent tensio-actif est choisi parmi les oléfines ($C_{14}$-$C_{16}$) sulfonates de sodium, le lauryl sulfate de triéthanolamine, le N-lauroylsarcosinate de sodium, un sel de potassium d'un condensat d'acides gras du coprah et d'une protéine hydrolysée, un éther de glucose et d'alcool décylique, un alkyl ($C_9$-$C_{18}$ linéaire ou ramifié) éther sulfate comportant 1 à 10 moles d'oxyde d'éthylène.

11. Composition selon l'une quelconque des revendications 1 à 10, caractérisée par le fait qu'elle contient des agents sequestrants.

12. Composition selon l'une quelconque des revendications 1 à 11, caractérisée par le fait qu'elle contient des adjuvants choisis parmi les agents nacrants et les silicones non volatiles, des agents de conditionnement de la peau, des extraits d'aloès ou de mauve, de l'huile de tournesol, du collagène, ou des colorants.

13. Composition selon l'une quelconque des revendications 1 à 12, caractérisée par le fait qu'elle contient des ingrédients actifs pour le traitement de la peau.

**Patentansprüche**

**Patentansprüche für folgende Vertragsstaaten : FR, BE, AT, GB, GR, IT, NL, DE, SE, CH, LI**

1. Zusammensetzung in Form einer festen Transparentseife, dadurch **gekennzeichnet**, daß sie eine Seife aus $C_{10}$-$C_{20}$-Fettsäuresalzen, ein Alkandiol-1,2 mit 10 bis 18 Kohlenstoffatomen, ein Transparenzmittel

und Wasser in Mengen unterhalb oder gleich 25 %, bezogen auf Gesamtgewicht der Zusammensetzung, enthält.

**2.** Zusammensetzung gemäß Anspruch 1, dadurch **gekennzeichnet**, daß das Alkandiol-1,2 10 bis 14 Kohlenstoffatome umfaßt.

**3.** Zusammensetzung gemäß Anspruch 1, dadurch **gekennzeichnet**, daß das Alkandiol-1,2 Dodekandiol-1,2 ist.

**4.** Zusammensetzung gemäß jedem der Ansprüche 1 bis 3, dadurch **gekennzeichnet**, daß das Alkandiol-1,2 in Mengenverhältnissen von 3 bis 10 vorzugsweise 4 bis 8 %, bezogen auf Gesamtgewicht der Zusammensetzung, vorliegt.

**5.** Zusammensetzung gemäß Anspruch 1, dadurch **gekennzeichnet**, daß die Seife aus Natriumsalzen von $C_{16}$-$C_{20}$-Fettsäuren oder aus Natriumsalzen von $C_{10}$-$C_{14}$-Fettsäuren oder deren Mischungen zusammengesetzt ist.

**6.** Zusammensetzung gemäß jedem der Ansprüche 1 bis 5, dadurch **gekennzeichnet**, daß die Seife in Mengenverhältnissen von 25 bis 40 Gew.-%, vorzugsweise 25 bis 35 %, bezogen auf Gesamtgewicht der Zusammensetzung, vorliegt.

**7.** Zusammensetzung gemäß Anspruch 5 oder 6, dadurch **gekennzeichnet**, daß die Seife aus 80 bis 90 Gew.-% Natriumsalzen von $C_{16}$-$C_{20}$-Fettsäuren und 10 bis 20 Gew.-% Natriumsalzen von $C_{10}$-$C_{14}$-Fettsäuren, bezogen auf Gesamtmenge an Seife, zusammengesetzt ist.

**8.** Zusammensetzung gemäß jedem der Ansprüche 1 bis 7, dadurch **gekennzeichnet**, daß das Transparenzmittel aus $C_2$-$C_6$-Polyolen, Harnstoff sowie deren Mischungen ausgewählt ist.

**9.** Zusammensetzung gemäß Anspruch 8, dadurch **gekennzeichnet** , daß das Polyol Glyzerin, Propylenglycol oder Sorbit ist, oder aus deren Mischungen zusammengesetzt ist.

**10.** Zusammensetzung gemäß jedem der Ansprüche 1 bis 9, dadurch **gekennzeichnet**, daß das Transparenzmittel in Mengenverhältnissen von 25 bis 50 Gew.-%, bezogen auf Gesamtgewicht der Zusammensetzung, vorliegt.

**11.** Zusammensetzung gemäß jedem der Ansprüche 1 bis 10, dadurch **gekennzeichnet**, daß sie enthält:
(i) 25 bis 40 Gew.-% einer Seife aus einer Mischung von Natriumsalzen von $C_{16}$-$C_{20}$-Fettsäuren und Natriumsalzen von $C_{10}$-$C_{14}$-Fettsäuren;
(ii) 3 bis 10 Gew.-% Dodecandiol-1,2;
(iii) 25 bis 50 Gew.-% eines $C_2$-$C_6$-Polyols oder von Harnstoff;
(iv) Wasser in Mengen unterhalb oder gleich 25 Gew.-%.

**12.** Zusammensetzung gemäß jedem der Ansprüche 1 bis 11, dadurch **gekennzeichnet**, daß sie zusätzlich ein synthetisches anionisches oder nicht-ionisches oberflächenaktives Mittel in Mengen von 1 bis 10 %, vorzugsweise 4 bis 8 %, bezogen auf Gesamtgewicht der Zusammensetzung, enthält.

**13.** Zusammensetzung gemäß Anspruch 12, dadurch **gekennzeichnet**, daß das oberflächenaktive Mittel ausgewählt ist aus dem Natriumsalz von $C_{14}$-$C_{16}$-Olefinsulfonaten, dem Laurylsulfat von Triethanolamin, dem Natriumsalz von N-Lauroylsarcosinat, einem Kaliumsalz eines Kondensats aus Fettsäuren von Kopra und eines hydrolysierten Proteins, einem Ether von Glucose und Decylalkohol, einem Natriumsalz eines linearen oder verzweigten $C_9$-$C_{18}$-Alkylethersulfats, enthaltend 1 bis 10 Mol Ethylenoxid.

**14.** Zusammensetzung gemäß jedem der Ansprüche 1 bis 11, dadurch **gekennzeichnet**, daß sie Sequestriermittel enthält.

**15.** Zusammensetzung gemäß jedem der Ansprüche 1 bis 14, dadurch **gekennzeichnet**, daß sie Hilfsstoffe enthält, ausgewählt aus Perlmutterglanz verleihenden Mitteln und nicht-flüchtigen Siliconen, Hautkonditioniermitteln, Extrakten von Aloe oder Malve, Sonnenblumenöl, Kollagen oder Färbemitteln.

**16.** Zusammensetzung gemäß jedem der Ansprüche 1 bis 15, dadurch **gekennzeichnet**, daß sie Wirkstoffe

zur Behandlung der Haut enthält.

17. Verwendung der Zusammensetzung gemäß der Ansprüche 1 bis 16 zum Waschen der Haut.

**Patentansprüche für folgenden Vertragsstaat : ES**

1. Zusammensetzung in Form einer festen Transparentseife, dadurch **gekennzeichnet** , daß sie enthält:
   (i) 25 bis 40 Gew.-%, vorzugsweise 25 bis 35 Gew.-%, bezogen auf Gesamtgewicht der Zusammensetzung, einer Seife aus $C_{10}$-$C_{20}$-Fettsäuresalzen,
   (ii) 3 bis 10 Gew.-%, vorzugsweise 4 bis 8 Gew.-%, eines Alkandiols-1,2 mit 10 bis 18 Kohlenstoffatomen,
   (iii) 25 bis 50 Gew.-% eines Transparenzmittels,
   (iv) wasser in Mengen unterhalb oder gleich 25 Gew.-%.

2. Zusammensetzung gemäß Anspruch 1, dadurch **gekennzeichnet**, daß das Alkandiol-1,2 10 bis 14 Kohlenstoffatome enthält.

3. Zusammensetzung gemäß Anspruch 1, dadurch **gekennzeichnet**, daß das Alkandiol-1,2 Dodecandiol-1,2 ist.

4. Zusammensetzung gemäß Anspruch 1, dadurch **gekennzeichnet**, daß die Seife aus Natriumsalzen von $C_{16}$-$C_{20}$-Fettsäuren oder Natriumsalzen von $C_{10}$-$C_{14}$-Fettsäuren oder deren Mischungen zusammengesetzt ist.

5. Zusammensetzung gemäß Anspruch 4, dadurch **gekennzeichnet**, daß die Seife aus 80 bis 90 Gew.-% Natriumsalzen von $C_{16}$-$C_{20}$-Fettsäuren und aus 10 bis 20 Gew.-% Natriumsalzen von $C_{10}$-$C_{14}$-Fettsäuren, bezogen auf Gesamtmenge an Seife, zusammengesetzt ist.

6. Zusammensetzung gemäß jedem der Ansprüche 1 bis 5, dadurch **gekennzeichnet**, daß das Transparenzmittel aus $C_2$-$C_6$-Polyolen, Harstoff sowie aus deren Mischungen ausgewählt ist.

7. Zusammensetzung gemäß Anspruch 6, dadurch **gekennzeichnet**, daß das Polyol Glycerin oder Propylenglycol oder Sorbit ist, oder aus deren Mischungen zusammengesetzt ist.

8. Zusammensetzung gemäß jedem der Ansprüche 1 bis 7, dadurch **gekennzeichnet**, daß sie enthält:
   (i) 25 bis 40 Gew.-% einer Seife aus einer Mischung von Natriumsalzen von $C_{16}$-$C_{20}$-Fettsäuren und Natriumsalzen von $C_{10}$-$C_{14}$-Fettsäuren;
   (ii) 3 bis 10 Gew.-% Dodecandiol-1,2;
   (iii) 25 bis 50 Gew.-% eines $C_2$-$C_6$-Polyols oder von Harnstoff;
   (iv) wasser in Mengen unterhalb oder gleich 25 Gew.-%.

9. Zusammensetzung gemäß jedem der Ansprüche 1 bis 8, dadurch **gekennzeichnet**, daß sie zusätzlich ein synthetisches anionisches oder nicht-ionisches oberflächenaktives Mittel in Mengen von 1 bis 10 %, vorzugsweise 4 bis 8 %, bezogen auf Gesamtgewicht der Zusammensetzung, enthält.

10. Zusammensetzung gemäß Anspruch 9, dadurch **gekennzeichnet**, daß das oberflächenaktive Mittel ausgewählt ist aus dem Natriumsalz von $C_{14}$-$C_{16}$-Olefinsulfonaten, dem Laurylsulfat von Triethanolamin, dem Natriumsalz von N-Lauroylsarcosinat, einem Kaliumsalz eines Kondensats aus Fettsäuren von Kopra und eines hydrolysierten Proteins, einem Ether von Glucose und Decylalkohol, einem linearen oder verzweigten $C_9$-$C_{18}$-Alkylethersulfat, enthaltend 1 bis 10 Mol Ethylenoxid.

11. Zusammensetzung gemäß jedem der Ansprüche 1 bis 10, dadurch **gekennzeichnet**, daß sie Sequestriermittel enthält.

12. Zusammensetzung gemäß jedem der Ansprüche 1 bis 11, dadurch **gekennzeichnet**, daß sie Hilfsstoffe enthält, ausgewählt aus Perlmutterglanz verleihenden Mitteln oder nicht-flüchtigen Siliconen, Hautkonditioniermitteln, Extrakten von Aloe oder Malve, Sonnenblumenöl, Kollagen oder aus Färbemitteln.

13. Zusammensetzung gemäß jedem der Ansprüche 1 bis 12, dadurch **gekennzeichnet**, daß sie Wirkstoffe zum Behandeln der Haut enthält.

## Claims

### Claims for the following Contracting States : FR, BE, AT, GB, GR, IT, NL, DE, SE, CH, LI

1. Composition in the form of a transparent, solid cake, characterized by the fact that it contains a soap consisting of salts of $C_{10}$-$C_{20}$ fatty acids, a 1,2-alkanediol containing from 10 to 18 carbon atoms, a transparency agent and water in quantities of less than or equal to 25% relative to the total weight of the composition.

2. Composition according to Claim 1, characterized in that the 1,2-alkanediol contains from 10 to 14 carbon atoms.

3. Composition according to Claim 1, characterized in that the 1,2-alkanediol is 1,2-dodecanediol.

4. Composition according to any one of Claims 1 to 3, characterized in that the 1,2-alkanediol is present in proportions between 3 and 10% and preferably between 4 and 8% relative to the total weight of the composition.

5. Composition according to Claim 1, characterized in that the soap consists of sodium salts of $C_{16}$-$C_{20}$ fatty acids or sodium salts of $C_{10}$-$C_{14}$ fatty acids or mixtures thereof.

6. Composition according to any one of Claims 1 to 5, characterized in that the soap is present in proportions of between 25 and 40% by weight relative to the total weight of the composition, and preferably between 25 and 35%.

7. Composition according to Claim 5 or 6, characterized in that the soap consists of 80 to 90% of sodium salts of $C_{16}$-$C_{20}$ fatty acids and of 10 to 20% of sodium salts of $C_{10}$-$C_{14}$ fatty acids by weight relative to the whole quantity of soap.

8. Composition according to any one of Claims 1 to 7, characterized in that the transparency agent is chosen from $C_2$-$C_6$ polyols, urea and mixtures of these.

9. Composition according to Claim 8, characterized in that the polyol is glycerine, propylene glycol or sorbitol or mixtures of these.

10. Composition according to any one of Claims 1 to 9, characterized in that the transparency agent is present in proportions of 25 to 50% by weight relative to the total weight of the composition.

11. Composition according to any one of Claims 1 to 10, characterized in that it contains:
    (i) 25 to 40% of a soap consisting of a mixture of sodium salts of $C_{16}$-$C_{20}$ fatty acids and of sodium salts of $C_{10}$-$C_{14}$ fatty acids;
    (ii) 3 to 10% of 1,2-dodecanediol;
    (iii) 25 to 50% of a $C_2$-$C_6$ polyol or of urea;
    (iv) water in proportions of less than or equal to 25% by weight.

12. Composition according to any one of Claims 1 to 11, characterized in that it additionally contains a synthetic anionic or nonionic surface-active agent in proportions of between 1 and 10% and preferably between 4 and 8% relative to the total weight of the composition.

**13.** Composition according to Claim 12, characteri-zed in that the surface-active agent is chosen from sodium ($C_{14}$-$C_{16}$)-olefinsulphonates, triethanolamine laurylsulphate, sodium N-lauroylsarcosinate, a potassium salt of a condensate of copra fatty acids and of a hydrolysed protein, an ether of glucose and of decyl alcohol, or a sodium (linear or branched $C_9$-$C_{18}$)-alkyl ether sulphate containing 1 to 10 moles of ethylene oxide.

**14.** Composition according to any one of Claims 1 to characterized in that it contains sequestering agents.

**15.** Composition according to any one of Claims 1 to 14, characterized in that it contains adjuvants chosen from pearlescent agents and nonvolatile silicones, skin conditioning agents, extracts of aloe or of mallow, sunflower oil, collagen or colorants.

**16.** Composition according to any one of Claims 1 to 15, characterized in that it contains active ingredients for the treatment of skin.

**17.** Use of the composition as claimed in Claims 1 to 16 for washing the skin.

**Claims for the following Contracting State : ES**

**1.** Composition in the form of a transparent, solid cake, characterized by the fact that it contains :
(i) 25 to 40 % by weight of a soap consisting of $C_{10}$-$C_{20}$ fatty acids and preferably between 25 to 35% by weight relative to the total weight of the composition,
(ii) 3 to 10% by weight of a 1,2-alkanediol containing from 10 to 18 carbon atoms and preferably 4 to 8% by weight,
(iii)25 to 50% by weight of a transparency agent,
(iv) water in quantities of less than or equal to 25% by weight.

**2.** Composition according to Claim 1 characterized in that the 1,2-alkanediol contains from 10 to 14 carbon atoms.

**3.** Composition according to Claim 1, characterized in that the 1,2-alkanediol is 1,2-dodecanediol.

**4.** Composition according to Claim 1, characterized in that the soap consists of sodium salts of $C_{16}$-$C_{20}$ fatty acids or sodium salts of $C_{10}$-$C_{14}$ fatty acids or mixtures thereof.

**5.** Composition according to Claim 4, characterized in that the soap consists of 80 to 90% of sodium salts of $C_{16}$-$C_{20}$ fatty acids and of 10 to 20% of sodium salts of $C_{10}$-$C_{14}$ fatty acids by weight relative to the whole quantity of soap.

**6.** Composition according to any one of Claims 1 to 5, characterized in that the transparency agent is chosen from $C_2$-$C_6$ polyols, urea or mixtures of these.

**7.** Composition according to Claim 6, characterized in that the polyol is glycerine, propylene glycol or sorbitol or mixtures of these.

**8.** Composition according to any one of Claims 1 to 7, characterized in that it contains :
(i) 25 to 40% of a soap consisting of a mixture of sodium salts of $C_{16}$-$C_{20}$ fatty acids and of sodium salts of $C_{10}$-$C_{14}$ fatty acids
(ii) 3 to 10% of 1,2-dodecanediol;
(iii) 25 to 50% of a $C_2$-$C_6$ polyol or of urea;
(iv) water in proportions of less than or equal to 25% by weight.

**9.** Composition according to any one of Claims 1 to 8 , characterized in that it additionally contains a synthetic anionic or nonionic surface-active agent in proportions of between 1 and 10% and preferably between 4 and 8% relative to the total weight of the composition.

**10.** Composition according to Claim 9, characterized in that the surface-active agent is chosen from sodium ($C_{14}$-$C_{16}$)-olefinsulfonates,triethanolaminelaurylsulphate , sodium N-lauroylsarcosinate, a potassium salt of a condensate of copra fatty acids and of a hydrolysed protein, an ether of glucose and of decyl alcohol, or a sodium (linear or branched $C_9$-$C_{18}$)-alkyl ether sulfate containing 1 to 10 moles of ethylene oxide.

**11.** Composition according to any one of Claims 1 to 10, characterized in that it contains sequestering agents.

**12.** Composition according to any one of Claims 1 to 11, characterized in that it contains adjuvants chosen from pearlescent agents and nonvolatile silicones, skin conditioning agents, extracts of aloe or of mallow, sunflower oil , collagen or colorants.

**13.** Composition according to any one of Claims 1 to 12, characterized in that it contains active ingredients for the treatment of skin.